# EUROPEAN PATENT APPLICATION

(11) **EP 4 324 339 A1**
(43) Date of publication of application: **21.02.2024**
(21) Application number: 23784876.7
(22) Date of filing: 16.03.2023
(51) Int. Cl.: A24B 13/00, A24B 15/14, A61K 31/465, A61K 9/00, A24B 15/30, A24B 15/32, A24B 3/14, A24B 13/02

(54) **SMOKING SUBSTANCE THAT ENABLES SMOKE SMOKING OR ORAL MIXED-USE AND PREPARATION METHOD THEREFOR**

(30) Priority: 05.04.2022 KR 20220042266
(71) Applicant: KT & G Corporation, Daejeon 34337 (KR)
(72) Inventor: JEONG, Jong Seong, Daejeon 34128 (KR); GO, Gyoung Min, Daejeon 34128 (KR); BAE, Hyung Jin, Daejeon 34128 (KR); SEO, Jangwon, Daejeon 34128 (KR); JANG, Chul Ho, Daejeon 34128 (KR); JUNG, Jinchul, Daejeon 34128 (KR)
(74) Representative: Ter Meer Steinmeister & Partner
(86) International application number: PCT/KR2023/003495
(87) International publication number: WO 2023/195648

(57) **Abstract**

The present disclosure relates to a smoking material for smoking with smoke or oral smoking, the smoking material including: glycerin included in an amount of 10 wt% to 40 wt% with respect to a total weight of the smoking material; a binder including methylcellulose and dextrin; and nicotine, and a method of manufacturing the same. The smoking material may be used for smoking with smoke and smokeless smoking through the oral cavity, and thus is advantageous in that the smoking is easily performed without the influence of environment.

## Description

### Technical Field

The present disclosure relates to a smoking material for smoking with smoke or oral smoking and a manufacturing method of the same.

### Background Art

Smoking articles are being proposed in a new form of being heated rather than being burned. Unlike conventional smoking articles, such non-combustible smoking articles are used by sucking an aerosol generated by heating a tobacco medium rather than burning the tobacco medium. One of such non-combustible smoking articles is a smoking article using electrical heating.

Meanwhile, smoking is generally performed by sucking an aerosol including nicotine, however, there is also a method of absorbing nicotine without smoke by placing a solid including nicotine in a mouth or chewing the solid.

A smokeless smoking product is a product that melts in the mouth to be absorbed through the mucous membrane, and helps the absorption of nicotine in the oral mucosa. Representative examples of smokeless tobacco include a nicotine patch, nicotine gum, a nicotine oral dissolving film (ODF), and the like.

In a case of the nicotine gum, nicotine is absorbed through the oral mucosa by placing the gum between the teeth and the cheek while chewing the gum slowly for about an hour. When the nicotine gum is used, an excessive amount of nicotine liberated and released at once may irritate the mouth and throat.

In a case of the nicotine patch, nicotine at a certain blood concentration is continuously supplied to the body, however the skin irritation may occur during long-term use.

The nicotine ODF is a formulation for absorbing nicotine by attaching the ODF to the oral cavity, such as on the tongue, the oral mucosa, or under the tongue and melting it. Such an ODF, as a sustained release agent, has the advantages of being able to be absorbed for a long period of time and being convenient to carry, but may cause a swallowing problem during the absorption due to its small size.

However, the conventional smoking articles have been provided in the form capable of limited smoking which is one of smoking with smoke, in which smoke is generated, or smokeless smoking. In this case, although a user had an article with smoking with smoke, if the smoking was not possible indoors, the use of the smoking article was restricted for a long period of time, or although the smoking with smoke became possible due to the movement of the user, if a smoking article carried by the user was in the form of a smokeless smoking article, only the smokeless smoking was possible.

### Prior Art Document

### Patent Document

Korean Patent Gazette No. 1998288

### Disclosure of the Invention

### Technical Goals

For this reason, an object of the present disclosure is to overcome the limitations of the prior art and to provide a smoking material capable of being used for smoking with smoke and smokeless smoking and a manufacturing method of the same, which may provide selectivity and convenience to users.

However, the technical goal obtainable from the present disclosure is not limited to the above-mentioned technical goal, and other unmentioned technical goals may be clearly understood from the following description by one of ordinary skill in the art to which the present disclosure pertains.

### Technical Solutions

The disclosure provides a smoking material for smoking with smoke or oral smoking, the smoking material including:
glycerin included in an amount of 10 wt% to 40 wt% with respect to a total weight of the smoking material;
a binder including methylcellulose and dextrin; and
nicotine.

In an implementation example of the present disclosure, the binder may include the methylcellulose and the dextrin at a weight ratio of 5:5 to 7:3.

In another implementation example of the present disclosure, the smoking material may be packaged in a pouch.

In still another implementation example of the present disclosure, the pouch may be a porous material.

In still another implementation example of the present disclosure, the pouch may be wrapped with a metal material.

In still another implementation example of the present disclosure, the smoking material may be in a form of beads, granules, or sheets.

In still another implementation example of the present disclosure, the smoking material may be used by being inserted into an oral cavity.

In still another implementation example of the present disclosure, the smoking material may be used by being inserted into an aerosol generating device.

In still another implementation example of the present disclosure, the smoking material may be porous.

Furthermore, the present disclosure provides a method of manufacturing a smoking material for smoking with smoke or oral smoking, the method including the following steps:
step S1 of manufacturing a fluid gel by adding glycerin and nicotine to a binder including methylcellulose and dextrin;
step S2 of manufacturing granules, beads, or sheets by processing the fluid gel; and
step S3 of packaging the granules, beads, or sheets in a pouch.

Moreover, the present disclosure provides an aerosol generating device into which the smoking material according to the present disclosure is inserted,
the device including:
a smoking material accommodation portion; and
a heater.

In another implementation example of the present disclosure, the device may further include a mouthpiece, and an upper cover, and a perforation may be formed on an outer circumferential surface of the upper cover.

### Effects

The smoking material of the present disclosure is capable of both smokeless smoking in which the smoking material is inserted into the oral cavity so that nicotine is absorbed into the skin, and smoking with smoke which is performed by inserting the smoking material into a heating-type aerosol generating device.

Depending on the environment at the time of smoking, a user may perform smoking with smoke by inserting the smoking material into a device and may also perform smokeless smoking by simply inserting the smoking material into the oral cavity. Therefore, it is advantageous that the smoking may be easily performed without the influence of the environment.

In addition, the method of manufacturing the smoking material includes a method of preparing a fluid gel including glycerin, a binder, and nicotine, and preparing it in the form of granules, beads, or sheets, and thus, the smoking material may be prepared in a simple way.

It should be understood that the effects of the present disclosure are not limited to the above-described effects, but are construed as including all effects that can be inferred from the configurations and features described in the following description or claims of the present disclosure.

### Brief Description of Drawings

FIG. 1 is a schematic diagram illustrating an example of a smoking material of the present disclosure.
FIG. 2 shows sheets produced by a method of manufacturing a smoking material of the present disclosure.
FIG. 3 shows a smoking material of the present disclosure in a packaged or wrapped form.
FIG. 4 shows an embodiment of a smoking material (a pouch) of the present disclosure to be inserted into an aerosol generating device in the present disclosure.
FIG. 5 shows a path of an airflow path formed in an aerosol generating device of the present disclosure.
FIG. 6 shows a result of comparing average temperatures of mainstream smokes of a pouch-type smoking article of the present disclosure and a heating-type cigarette P2 of the related art.

### Best Mode for Carrying Out the Invention

Hereinafter, embodiments will be described in detail with reference to the accompanying drawings. However, various alterations and modifications may be made to the embodiments. Here, the embodiments are not construed as limited to the disclosure. The embodiments should be understood to include all changes, equivalents, and replacements within the idea and the technical scope of the disclosure.

The terminology used herein is for the purpose of describing particular embodiments only and is not to be limiting of the embodiments. The singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, like reference numerals refer to like components and a repeated description related thereto will be omitted. In the description of embodiments, detailed description of well-known related structures or functions will be omitted when it is deemed that such description will cause ambiguous interpretation of the present disclosure.

The term "with smoke" used herein means that smoke (an aerosol) is generated, that is, "smoking with smoke" refers to smoking in which smoke is generated when a user smokes. For example, it may refer to smoking performed by a user sucking smoke generated by inserting a smoking material (a smoking medium) to an aerosol generating device.

The term "smokeless" used herein means that smoke (an aerosol) is not generated, that is, "smokeless smoking" refers to smoking in which smoke is not generated when a user smokes. For example, it may refer to smoking performed by absorbing nicotine through the skin in the oral cavity by putting the smoking material into the oral cavity.

The term "granules or beads" used herein refer to a material in the form of round and fine grains, which are substantially spherical grains having an average diameter relatively larger than that of powder.

The term "porous" used herein refers to a state in which a solid has pores (gaps) inside or on a surface thereof.

The present disclosure relates to a smoking material for smoking with smoke or oral smoking, the smoking material including: glycerin included in an amount of 10 to 40 wt% with respect to a total weight of the smoking material; a binder including methylcellulose and dextrin; and nicotine. As described above, the smoking material of the present disclosure may be used for the smoking with smoke in which the smoking material is inserted into an aerosol generating device to generate an aerosol, and the smokeless smoking performed by inserting the smoking material into the oral cavity.

According to an embodiment of the present disclosure, the binder includes methylcellulose and dextrin at a weight ratio of 5:5 to 7:3. The methylcellulose and the dextrin may be included desirably at a weight ratio of 5.5:4.5 to 6.5:3.5 and may be mixed more desirably at a weight ratio of 6:4.

The content of the dextrin in the binder needs to be smaller than the content of the methylcellulose, and if the content of the dextrin is greater than the content of the methylcellulose, wrinkles may be formed on a sheet, making the processing difficult. In addition, when the methylcellulose is included in an amount of 70 wt% or more with respect to 100 wt% of the binder, the hardness of a sheet may be reduced, making it difficult to cut or slice the sheet into granules or beads.

The binder may be included in an amount of 30 wt% to 70 wt% with respect to a total weight of the smoking material. When the binder is included in an amount less than 30 wt%, bonding strength sufficient to produce a sheet may not be obtained, and when the binder is included in an amount more than 70 wt%, the hardness of a sheet may be excessively increased, affecting processability of cutting or slicing the sheet.

The binder may further include, in addition to the methylcellulose and the dextrin, one or more binders selected from the group consisting of carboxymethylcellulose, starch, whole charcoal gum, gum arabic, guar gum, pectin, gelatin, agar, pullulan, mannitol, and xylitol, in consideration of physical properties of the smoking material to be manufactured, and the binder is not limited to the materials described above.

In the present disclosure, the glycerin is included in an amount of 10 wt% to 40 wt% with respect to the total weight of the smoking material and may be included more desirably in an amount of 12 wt% to 40 wt%. When the glycerin is included in an amount less than 12 wt%, sufficient atomization may not occur, making it difficult to perform the smoking with smoke, and when the glycerin is included in an amount more than 40 wt%, an excessive amount of atomization may be generated. In addition, the smoking material may further include, in addition to the glycerin, at least one of propylene glycol, ethylene glycol, dipropylene glycol, diethylene glycol, triethylene glycol, or tetraethylene glycol.

In the present disclosure, the nicotine may be included in an amount of 0.1 wt% to 8 wt% with respect to the total weight of the smoking material, however, the content of the nicotine may be appropriately changed in consideration of taste, flavor, or throat hit of the smoking material to be manufactured. The smoking material of the present disclosure may not substantially include solid materials based on tobacco raw materials such as plate-shaped leaf tobacco, cut tobacco, or reconstituted tobacco raw materials. That is, the smoking material may include only nicotine without materials derived from tobacco plants.

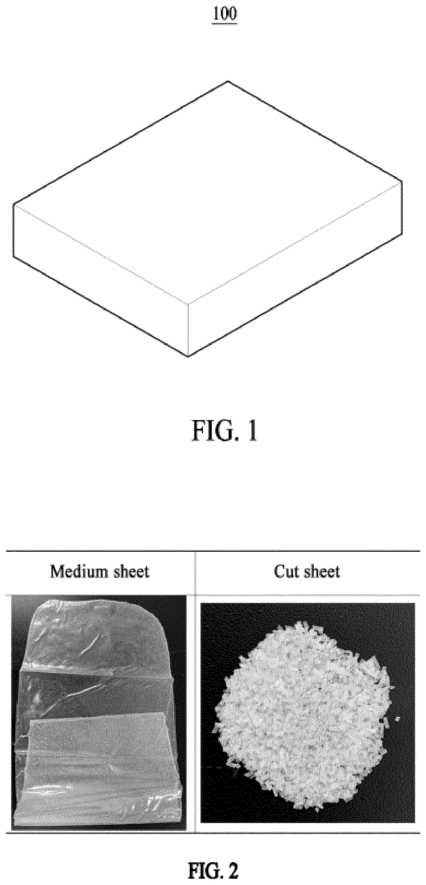

According to an embodiment of the present disclosure, the smoking material is desirably in the form of granules, beads, or sheets. In an example, the smoking material may be in the form of a sheet as shown in FIG. 1. The smoking material of the present disclosure is manufactured in the form of granules, beads, or sheets by processing a fluid gel including glycerin, a binder, and nicotine.

In the present disclosure, there is no limitation to a method of processing the fluid gel into the form of granules or beads. The processing of the fluid gel into the form of granules or beads may be performed by a dry granulation method or a spray drying method of compressing a fluid gel to obtain a large piece thereof and pulverizing it into granules, or may be performed through an electrostatic spraying device using an electrostatic charge method.

In another example, the fluid gel may be applied onto any substrate with a thickness of 0.8 millimeters (mm) or more and dried to manufacture an original sheet (left side of FIG. 2), and the original sheet itself may be sliced or cut into a certain size to manufacture sheets (right side of FIG. 2).

In an embodiment of the present disclosure, the granules or beads may have an average diameter of 0.5 mm or more. The granules or beads desirably have an average diameter of 1 mm or more, and more desirably have an average diameter of 1 mm to 3 mm. When the average diameter of the granules or beads is less than 0.5 mm, a powder may be sucked into the nasal or oral cavity, unlike the smoking material of the present disclosure that may be used for the smokeless smoking or the smoking with smoke.

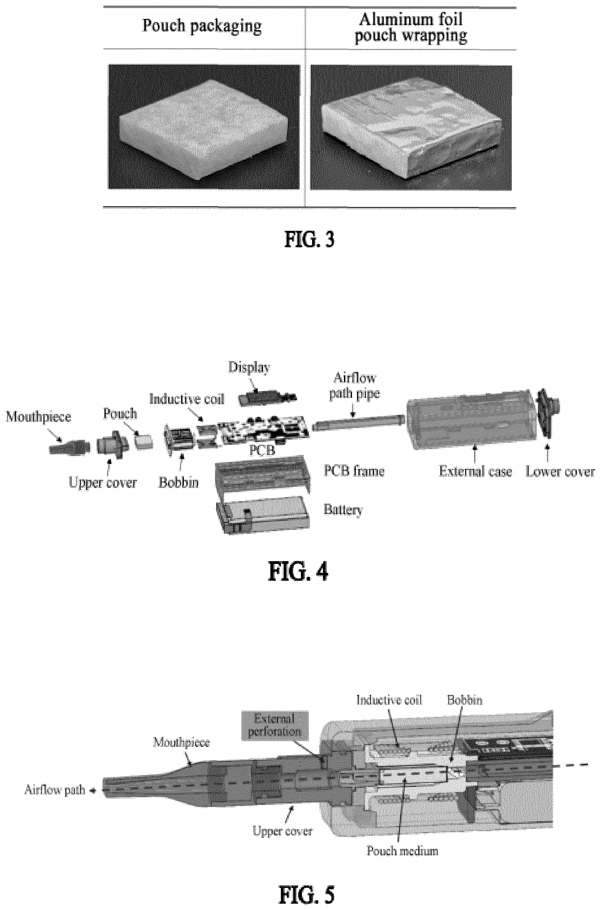

In an embodiment of the present disclosure, as shown in an example on the left side of FIG. 3, the smoking material may be packaged in a pouch. The smoking material in the form of granules, beads, or sheets may be provided to a user in a packaged form surrounded by a pouch material, and the pouch may include a porous material.

In an embodiment of the present disclosure, the pouch may have a porosity of 30% to 70%. The porosity refers to a volume ratio of a substrate constituting the porous material and pores existing in the substrate. When the porosity is less than 30%, nicotine may not be easily transferred from the granules or beads in the pouch due to insufficient porosity, and when the porosity exceeds 70%, the granules or beads may leak out.

In addition, the pores formed in the pouch desirably have an average diameter of 30 µm or more, and may be changed according to the size of granules or beads in the pouch, however, the pores may more desirably have a pore diameter of 30 µm to 100 µm. When the average pore size is less than 30 µm, nicotine may not be easily transferred from the granules or beads in the pouch due to insufficient porosity, and when the average pore size exceeds 100 µm, the physical properties of the granules or beads may be affected.

In addition, the pouch is formed of a material having heat resistance and water resistance. The pouch may include a material having heat resistance so that the pouch is not damaged even when the smoking material is inserted into a heating-type device, and having water resistance so that the pouch is not damaged by saliva in the mouth or weak chewing even when it is inserted into the oral cavity.

The pouch may be made of a material including any one of porous paper, nonwoven fabric, or polymer material. The pouch completely encloses the smoking material, and may be substantially sealed by heat bonding, pressure bonding, ultrasonic bonding, or the like, if necessary, and the bonding method is not limited.

A basis weight of the porous material forming the pouch is desirably 10 g/m² or more. More desirably, a porous material having a basis weight of 10 g/m² to 50 g/m² may be used. When the basis weight of the pouch is less than 10 g/m², the strength of the pouch may decrease, and when the basis weight thereof exceeds 50 g/m², a thickness of the pouch may affect the smooth transfer of the smoking material inside the pouch.

According to an embodiment of the present disclosure, the smoking article may have a rectangular parallelepiped shape with a square top and bottom cross section, having a height of about 1 mm to 10 mm and horizontal and vertical lengths of 8 to 30 mm. When the height is less than 1 mm, there is a risk of being swallowed due to the small thickness, and when the height exceeds 10 mm, it may cause discomfort in positioning in the oral cavity. In addition, when the horizontal and vertical lengths are less than 8 mm, there is a risk of being swallowed due to the small size, and when the horizontal and vertical lengths exceed 30 mm, it may cause discomfort in positioning in the oral cavity. The height and the horizontal and vertical lengths may include a thickness of the pouch itself.

However, this is an example, and the cross section of the smoking article may have any shape such as a rectangle, a circle, a star shape, a heart shape, or other polygons.

According to an embodiment of the present disclosure, the smoking material may have a weight of 200 to 500 mg. More desirably, when the smoking material has a weight range of 300 to 350 mg, 14 puffs or more atomization may be occur when performing the smoking with smoke.

As described above, the smoking material may be inserted into an aerosol generating device to be used for the "smoking with smoke", or may be inserted into the oral cavity to be used for the "oral smoking", and therefore the user may use the smoking material by selecting the smoking method according to the user's needs.

For the aerosol generating device, any device may be used as long as it is an external heating-type device, and the device may include a smoking material accommodation portion for providing a space for accommodating a smoking material; and a heater.

Although FIG. 4 shows an example of the type of the aerosol generating device, it is clear that the aerosol generating device is not limited as long as it is a heating-type aerosol generating device having a predetermined space into which a smoking material may be inserted.

The term "aerosol generating device" used herein refers to a device that generates an aerosol using a smoking material such that the aerosol may be inhaled through the mouth of a user directly to the lungs of the user.

In an implementation example of the present disclosure, referring to FIG. 4, the device may further include a mouthpiece; and an upper cover coupled to the mouthpiece.

In an embodiment of the present disclosure, a perforation may be formed on an outer circumferential surface of the upper cover. As shown in FIG. 5, the external perforation may be connected to an airflow path connected from an airflow pass pipe to the mouthpiece through a pouch medium, and the external perforation may improve the heat of mainstream smoke and increase the amount of atomization during smoking.

In an embodiment of the present disclosure, referring to FIG. 4, the device may further include the airflow pass pipe connected to the smoking material accommodation portion; a battery for providing power to the device; and an inductive coil formed at a position spaced apart from the smoking material accommodation portion.

In an embodiment of the present disclosure, the smoking material accommodation portion and the inductive coil may be spaced apart from each other by 1.5 to 3.5 mm. When the inductive coil is positioned at a distance less than 1.5 mm from the smoking material accommodation portion, the pouch may overheat because the inductive coil and the smoking material are close to each other, and when the inductive coil is positioned at a distance exceeding 3.5 mm from the smoking material accommodation portion, heat may not be smoothly transferred.

The device may further include additional components required for the operations of the device, in addition to the components described above. For example, the device may include a display for checking a state of the device, and a controller for controlling the battery to provide power, and may further include an external case and a lower cover for housing components, such as the smoking material accommodation portion, the battery, and the airflow path pipe, and the like.

In addition, in an embodiment of the present disclosure, the smokeless smoking is performed by inserting a smoking material into the oral cavity. More specifically, the smokeless smoking may be performed by placing a smoking material between the lips and gums and then absorbing nicotine included in the smoking material through the skin inside the mouth (oral mucosa). When the absorption of the nicotine is completed, the smoking material may be discharged from the mouth to the outside.

In an embodiment of the present disclosure, the smoking material may be packaged in a pouch and then wrapped with a metal material. When the pouch is wrapped with a metal material, the metal material itself may be heated (induction heating) for the smoking, without additionally providing a heating material such as a susceptor in the aerosol generating device. If the smoking material wrapped with the metal material is desired to be used for the smokeless smoking, the smoking material packaged in the pouch may be used after peeling the metal material off.

The metal material may include one or more types of metal selected from the group consisting of aluminum (Al), chromium (Cr), cobalt (Co), molybdenum (Mo), copper (Cu), stainless steel (SUS), and nickel (Ni), and the metal material may be an alloy material including two or more types of metal materials. The metal material may desirably include an aluminum material.

The metal material may be in the form of a thin film and may be provided in the form of being coated on the pouch, if necessary.

In another embodiment of the present disclosure, when the smoking with smoke is performed with the smoking material packaged in only the pouch without the wrapping with the metal material, the smoking may be performed by heating the smoking material through a susceptor using an aerosol generating device including the susceptor.

In addition, the present disclosure provides a method of manufacturing a smoking material for smoking with smoke or oral smoking, the method including the following steps:
step S1 of manufacturing a fluid gel by adding and mixing glycerin and nicotine to a binder including methylcellulose and dextrin;
step S2 of manufacturing granules, beads, or sheets by processing the fluid gel; and
step S3 of packaging the sheets in a pouch.

In another embodiment of the present disclosure, the manufacturing method of the present disclosure will be described in more detail for each step. A smoking material manufactured by the manufacturing method described above is substantially the same as the smoking material described above, and therefore, it is to be understood that some descriptions of the manufacturing method are not duplicated and are omitted, because they are included in the descriptions of the smoking material described above.

In an embodiment of the present disclosure, step S1 herein is a step of manufacturing a fluid gel by adding and mixing glycerin and nicotine to a binder including methylcellulose and dextrin. An appropriate solvent may be further added so that the components of step S1 are easily mixed and a fluid gel state is obtained. The appropriate solvent may be water or ethanol. The solvent may evaporate during a drying process of the fluid gel and thus may not be included in a final smoking material.

In an embodiment of the present disclosure, step S2 herein is a step of manufacturing a sheet formed of granules or beads by processing the fluid gel manufactured in step S1. In step S2, as described above, the fluid gel may be processed into granules or beads by the granulation method or the like, and then the granules or beads may be aggregated or arranged into a sheet to manufacture a sheet, or the fluid gel may be applied onto a substrate and dried, and a sheet obtained by slicing or cutting the substrate may be used as it is.

In an embodiment of the present disclosure, step S3 herein is a step of packaging the manufactured sheet in a pouch. A packaging method of the sheet is not limited as long as it is a packaging method generally used in the manufacturing process of a smoking article or a smoking material.

In another implementation example of the present disclosure, the smoking material of the present disclosure may further include components, such as a flavoring material and other additives that may be included in a smoking product in general.

As the flavoring material, all materials capable of imparting flavor and/or aroma characteristics or enhancing an aromatic taste of smoking and applicable to a smoking article may be used without limitation. The flavoring material may include, for example, at least one selected from a group consisting of an organic acid, such as a lactic acid, a citric acid, a malic acid, and the like, licorice, sucrose, fructose syrup, isosweet, cocoa, lavender, cinnamon, cardamom, celery, fenugreek, cascarilla, white sandalwood, bergamot, geranium, honey essence, rose oil, vanilla, lemon oil, orange oil, mint oil, cinnamon, caraway, cognac, jasmine, chamomile, menthol, cassia, ylang-ylang, salvia, spearmint, ginger, cilantro, a clove extract (or a clove material), and coffee, but is not limited thereto.

Hereinafter, embodiments will be described in more detail. However, various alterations and modifications may be made to the embodiments. Here, the embodiments are not construed as limited to the disclosure. The embodiments should be understood to include all changes, equivalents, and replacements within the idea and the technical scope of the disclosure.

### 1. Manufacture of Smoking Material

According to a composition of Table 1 below, a smoking material was prepared.

**[Table 1]**

| Methylcellulose | Dextrin | Glycerin | Nicotine | Ethyl alcohol | Water |
|---|---|---|---|---|---|
| 38.3% | 25.5% | 35% | 1.2% | 180 g | 20 g |

The methylcellulose, dextrin, glycerin, nicotine, ethyl alcohol, and water were mixed with each other to manufacture a fluid gel. The manufactured gel was applied onto a substrate to manufacture a sheet, and the sheet was cut to manufacture granules. The manufactured granules were wrapped with a nonwoven fabric pouch to complete a smoking material. After the completion, the pouch was wrapped once more with an aluminum film.

### 2. Heating of Smoking Material

The manufactured smoking material was combined with a device having the configuration shown in FIG. 3, preheated with a current of 2300 mAh for 30 seconds, primarily heated with a current of 1500 mAh for 30 seconds, and then heated with a current of 1200 mAh for 220 seconds. Details for the operation of the device are shown in Table 2 below (spacing distance: distance between a smoking material and an inductive coil).

**Table 2]**

| Coil L value | Coil R value | Resonance Cap | Control frequency | Spacing distance | Thickness of aluminum foil |
|---|---|---|---|---|---|
| 3.63 uH | 23 mΩ | 88 nF | 400 khz | 2.2 mm | 18 um |

### 3. Analysis of smoke components of smoking material

Characteristics of smoke components transferred through the heating of the smoking material (a pouch medium) of the present disclosure were confirmed, and results of comparison with a conventional heating-type cigarette P2 were shown in Table 3 below.

**[Table 3]**

| Product Name | | TPM | Tar | Nicotine | PG | Gly | Moisture |
|---|---|---|---|---|---|---|---|
| Pouch medium (Preheat for 30s) | AVE (mg/stick) | 54.0 | 43.3 | 0.41 | < LOQ | 24.3 | 10.3 |
| | SD (mg/stick) | 2.3 | 1.6 | 0.0 | | 0.5 | 0.7 |
| | CV (%) | 4.3 | 3.6 | 9.3 | | 1.9 | 6.8 |
| P2 | AVE (mg/stick) | 52.4 | 27.5 | 0.47 | 2.7 | 10.5 | 24.5 |
| | SD (mg/stick) | 1.9 | 2.0 | 0.0 | 0.2 | 1.1 | 0.8 |
| | CV (%) | 3.6 | 7.2 | 8.4 | 8.9 | 10.0 | 3.2 |

| | | | | | | | |
|---|---|---|---|---|---|---|---|
| *Smoking conditions: the pouch medium (55 ml, 2 sec, 30 sec, and 8 puffs), P2 (55 ml, 3 sec, 30 sec, and 9 puffs) | | | | | | | |

As a result, it was confirmed that, in the smoking material of the present disclosure, the amount of moisture transfer was lower and the amount of glycerin transfer was 2 times higher than those of P2. In addition, the amount of nicotine transfer was at a similar level as that of P2.

In addition, it was confirmed that the smoking material of the present disclosure had a large amount of atomization and excellent atomization persistency with substantially no irritation, even though it is a product including no filter, compared to P2. In addition, a light caramel flavor was confirmed due to the influence of dextrin.

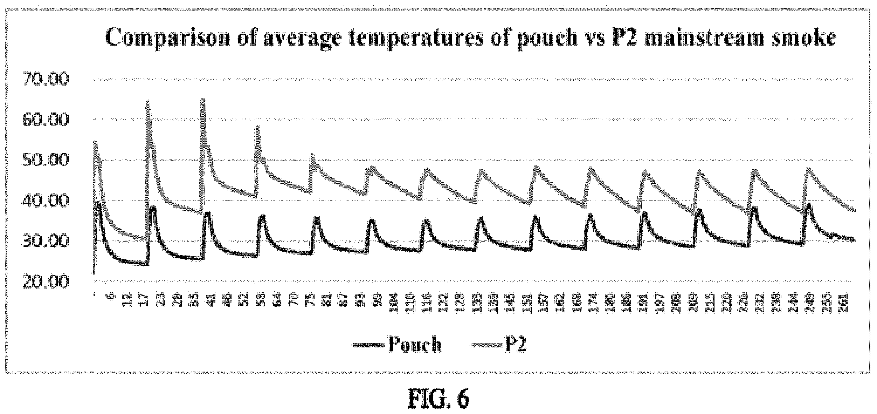

A result of comparing mainstream smoke temperatures of the P2 and the smoking material of the present disclosure is shown in FIG. 6. As shown in FIG. 6, it was confirmed that an average of maximum temperatures of the mainstream smoke was lower than that of P2 (65°C) by about 25°C, and it was confirmed that the perforation formed on the upper cover effectively lowered the temperature of the mainstream smoke (based on an average temperature obtained from four times of measurement).

While the embodiments are described with reference to drawings, it will be apparent to one of ordinary skill in the art that various alterations and modifications in form and details may be made in these embodiments without departing from the spirit and scope of the claims and their equivalents. For example, suitable results may be achieved if the described techniques are performed in a different order and/or if components in a described system, architecture, device, or circuit are combined in a different manner and/or replaced or supplemented by other components or their equivalents.

Therefore, other implementations, other embodiments, and equivalents to the claims are also within the scope of the following claims.

### Explanation of Reference Numerals

100: Smoking material

### Industrial availability

The smoking material of the present disclosure may be used for both of the smokeless smoking in which the smoking material is inserted into the oral cavity and nicotine is absorbed into the skin, and the smoking with smoke performed by inserting the smoking material into a heating-type aerosol generating device. The smoking material is advantageous in that the smoking is easily performed without the influence of environment, and thus it may be usefully used in the field of smoking product industry.

## Claims

1. A smoking material for smoking with smoke or oral smoking, the smoking material comprising:
glycerin included in an amount of 10 wt% to 40 wt% with respect to a total weight of the smoking material;
a binder comprising methylcellulose and dextrin; and
nicotine.

2. The smoking material of claim 1, wherein the binder comprises the methylcellulose and the dextrin at a weight ratio of 5:5 to 7:3.

3. The smoking material of claim 1, wherein the smoking material is packaged in a pouch.

4. The smoking material of claim 3, wherein the pouch is a porous material.

5. The smoking material of claim 3, wherein the pouch is wrapped with a metal material.

6. The smoking material of one of claims 1 to 3, wherein the smoking material is in a form of beads, granules, or sheets.

7. The smoking material of one of claims 1 to 3, wherein the smoking material is used by being inserted into an oral cavity.

8. The smoking material of one of claims 1 to 3, wherein the smoking material is used by being inserted into an aerosol generating device.

9. A method of manufacturing a smoking material for smoking with smoke or oral smoking, the method comprising:
step S1 of manufacturing a fluid gel by adding glycerin and nicotine to a binder comprising methylcellulose and dextrin;
step S2 of manufacturing granules, beads, or sheets by processing the fluid gel; and
step S3 of packaging the granules, beads, or sheets in a pouch.

10. The method of claim 9, wherein the binder comprises the methylcellulose and the dextrin at a weight ratio of 5:5 to 7:3.

11. The method of claim 9, wherein the pouch is a porous material.

12. The method of claim 9, wherein the pouch is wrapped with a metal material.

13. An aerosol generating device into which the smoking material of claim 1 is inserted, the aerosol generating device comprising:
a smoking material accommodation portion; and
a heater.

14. The aerosol generating device of claim 13, further comprising:
a mouthpiece; and
an upper cover,
wherein a perforation is formed on an outer circumferential surface of the upper cover.
